# EUROPEAN PATENT APPLICATION

(11) **EP 0 953 319 A2**
(43) Date of publication of application: **03.11.1999**
(21) Application number: 99303320.8
(22) Date of filing: 28.04.1999
(51) Int. Cl.: A61F 2/06

(54) **Jawed crimping tool and method of use**

(30) Priority: 28.04.1998 US 69011
(71) Applicant: Advanced Cardiovascular Systems, Inc., Santa Clara, California 95054 (US)
(72) Inventor: Morales, Stephen A., Santa Clara, California 95050 (US)
(74) Representative: McLeish, Nicholas Alistair Maxwell

(57) **Abstract**

A stent crimping tool (22) for firmly and uniformly crimping a conventional or radioactive stent (10) onto a balloon catheter (11) is constructed from a housing having a proximal section (24) rotatably connected to a distal section (26). A cylindrical cavity (40) having a tapered end (42) is formed into the proximal section (24). Inside the cavity is affixed a transparent cylindrical collar (32) having radial slots (66) leading to a central passage (70) extending along its axis, and a conical end (64) that fits into the tapered end (42). Teeth (30) made of trapezoidally-shaped flat plates each having an angular proximal edge (58) and a radiused edge (62) slide into their respective slots (66) in the collar (32). A transparent screw-feed (28) having a hollow core (54) and a slotted head (50) that receives the radiused edge (62) of each tooth (30) is threaded to the distal section (26). A balloon catheter (11) is passed through a passage (48) in the proximal section (24) and the collar (32) and an uncrimped stent (10) positioned in the hollow core (54) of the screw a loaded thereon. Rotating the distal section (26) of the housing advances the screw and teeth (30) towards the tapered cavity, which has angled walls that force the teeth to converge radially inward. This convergence causes the radiused edges (62) of the teeth (30) to collectively crimp the stent (10) onto the balloon catheter (11).

## Description

### BACKGROUND OF THE INVENTION

The present invention relates to an apparatus [or loading a tubular graft, such as a stent, onto the distal end of a catheter assembly of the kind used, for example, in percutaneous transluminal coronary angioplasty (PTCA) or percutaneous transluminal angioplasty (PTA) procedures.

In a typical PTCA procedure, a guiding catheter is introduced percutaneously into the cardiovascular system of a patient through the brachial or femoral arteries and then is advanced through the vasculature until the distal end of the guiding catheter is in the ostium. A guide wire and a dilation catheter having a balloon on the distal end next are introduced through the guiding catheter, with the guide wire sliding within the dilatation catheter. The guide wire first is advanced out of the guiding catheter inot the patient's coronary vasculature and the dilatation catheter then is advanced over the previously advanced guide wire until the dilatation balloon is properly positioned across the arterial lesion. Once in position across the lesion, a flexible and expandable balloon is inflated to a predetermined size with a radiopaque liquid at relatively high pressures to radially compress the atherosclerotic plaque of the lesion against the inside of the artery wall and thereby dilate the lumen of the artery. The balloon then is deflated to a small profile so that the dilatation catheter can be withdrawn from the vasculature of the patient and the blood flow resumed through the dilated artery. As should be appreciated by those skilled in the art, while the above-described procedure is typical, it is not the only method used in angioplasty.

In angioplasty procedures of the kind referenced above, restenosis of the artery may develop over time, which may require another angioplasty procedure, a surgical bypass operation, or some other method of repairing or strengthening the area. To reduce the likelihood of the development of restenosis and to strengthen the area, a physician can implant an intravascular prosthesis for maintaining vascular patency, commonly known as a stent, inside the artery at the lesion. The stent is crimped tightly onto the balloon portion of the catheter and transported in its delivery diameter through the vasculature of the patient. At the deployment site, the stent is expanded to a larger diameter, often by inflating the balloon portion of the catheter. The stcnt also may be of the self-expanding type.

Because the catheter and stent travel through the vasculature of the patient, and probably through the coronary arteries, the stent must have a small delivery diameter and must be attached firmly to the catether until the physician is ready to implant it. Thus, the stent must be loaded onto the catheter so that the stent does not interfere with delivery, and the stent must not come off the catheter until the stent is implanted.

In procedures in which the stent is placed over the balloon portion of the catheter, it is necessary to crimp the stent onto the balloon portion to reduce the diameter of the stent and to prevent it from sliding off the catheter when the catheter is advanced through the vasculature of the patient. Non-uniform crimping can result in sharp edges being formed along the crimped stent which can exhibit an eneven surface upon camping. Further, non-uniform stent crimping may not achieve the desired minimal profile for the stent-and-catheter assembly. When the stent is not crimped reliably onto the catheter, the stent may slide off the catheter and into the vasculature of the patient prematurely as a loose foreign body, possibly causing blood clots in the vasculature, including thrombosis. Therefore, it is important to insure that a stent properly is crimped onto a catheter in a uniform and reliable manner.

This crimping often is accomplisbed manually, which can be unsatisfactory due to the uneven application of force by the hands. Non-uniform or loosely applied crimps result. In addition, it is difficult to judge visually when a uniform and reliable crimp has been applied.

Some stents of the self-expanding type are difficult to load by hand onto a delivery device such as a catheter. Further, the longer the period of time the stent is handled the higher is the likelihood that human error will adversely affect the crimping process. Accordingly, there is a need in the art for a device which can be use to crimp a stent reliably onto a catheter.

Attempts have been to devise a tool for crimping a stent onto a balloon delivery catheter. An example of such a tool comprises a series of plates having substantially flat and parallel surfaces that move in a rectilinear fashion with respect to each other. A catheter onto which a stent has been overlaid is disposed between these flat and parallel surfaces, which surfaces crimp the stent onto the outside of the catheter when the surfaces are moved relative to each other while pressure simultaneously is applied. The plates have multiple degrees of freedom and may have force-indicating transducers to measure and indicate the force applied to the catheter during crimping of the stent.

Another stent-loading tool design is comprised of a tubular member housing a bladder. The tubular member and bladder are constructed to old a stent that is to be crimped onto a balloon catheter assembly. Upon placement of the stent over the balloon portion of the catheter, a valve in the loading tool is activated to inflate the bladder. The bladder compresses the stent radially inward to a reduced diameter onto the balloon portion of the catheter to achieve a snug fit. In this way, the stent is crimped onto the distal end of a balloon catheter with a minimum of human handling. The foregoing stent crimping tools are disclosed in, for example, U.S. Patent Nos. 5,437,083 and 5,546,646 to Williams et al.

Yet another stent crimping tool has been manufactured under the tradename BARD XT, by C.R. Bard, Inc. which, more appropriately, is characterized as a stent loader. This device is constructed from a rigid, tubular body with a ball at one end connected to a plurality of long, thin strips passing through the tubular body. An uncrimped stent is placed over the plurality of long, thin strips, which hold the stent in an expanded state. The balloon portion of a catheter is inserted into the cylindrical space formed by the plurality of strips. When the user pulls the ball while holding the tubular body against the stent, the strips are slid from beneath the stent and the stent is transferred onto the balloon portion.

Still another conventional stent crimping tool is manufactured by Johnson & Johnson and appears similar to a hinged nutcracker. More particularly, the tool is comprised of two hand-operated levers that an hinged at one end and gripped in the palm of the hand at the opposite end. A cylindrical opening, holding a crimping tube, is provided through the mid-portion of the tool, and is adapted to receive therein a stent loaded onto a balloon catheter. The crimping operation is accomplished when the user squeezes the handle, thereby applying pressure to the crimping tube, which tube in turn pinches the stent onto the balloon catheter.

While the prior art devices are suitable for crimping stents onto balloon catheters, these devices can be associated with problems attributable to non-uniform crimping forces, which result in non-uniform crimps. Consequently, these devices are not suitable for use by physicians in a catheterization laboratory who are charged with crimping the stent onto a balloon catheter prior to performing a procedure in a patient.

### SUMMARY OF THE INVENTION

Both PTCA and PTA procedures have become commonplace in treating stenoses or lesions in blood vessels and coronary arteries. In approximately 35% to 40% of the procedures, restenosís may develop which índicates a further procedure, such as, angioplasty, an atherectomy or a bypass operation to restore the patency of the vessel. Intravascular stents now are being deployed after PTCA and PTA procedures, and after atherectomies, in order to thwart restenosis. Importantly, such stents, after being mounted on the balloon portion of a catheter, must be tightly crimped there on to provide a low profile delivery diameter, and to insure that the stent stays positioned on the balloon until the balloon is expanded and the stent is deployed into the vessel at the target area. Embodiments of the invention are directed to a crimping tool that repeatedly can provide a uniform and tight crimp to insure the low-profile diameter of the stent on the balloon portion of the catheter, and to insure that the stent remains attached firmly until it is implanted in the vessel by expanding the balloon.

In a preferred embodiment, there is provided a tool for crimping a stent onto a balloon catheter, comprising a housing including a proximal section and a distal section, wherein the proximal section includes a cylindrical cavity having a tapered end, and an opening in communicaton with the cavity, a cylindrical collar having a conical end conforming to the tapered end when the collar is positioned therein, wherein the collar further includes a plurality of slots extending radially and long a length thereof and intersecting at the conical end, and having a passage therethrough that is in communication with the opening in the proximal section, and a plurality of teeth having a plate shape, each tooth or plate having an angular proximal edge, and being slidably disposed inside a corresponding slot in the collar, each plate having a radiused edge extending into the passage, and a screw-feed extending into the passage, wherein the screw-feed includes radial slots that lead into a hollow core, and wherein the slot receive the respective radiused edges of the plates extending therein, and wherein the screw-feed is threaded to the distal section of the housing.

In use, an uncrimped stent is loaded manually into the hollow core of the tool. At the opposite end of the tool, the balloon catheter is inserted through the opening into the cylindrical cavity at the proximal section of the housing, which leads into the hollow core of the screw which holds the uncrimped stent.

Ideally, the tool is held in the dominant hand of the user, while the user's other hand advances a balloon catheter into the hollow core holding the stent. In a preferred embodiment, the crimping tool is made from a transparent or translucent material so that the user can observe and track the process of inserting the balloon catheter. Looking into the hollow core of the screw-feed, the user manually aligns and loads the uncrimped stent onto the balloon catheter.

Alternatively, a tool embodying the invention can be advanced over a guide wire. As a second step, the balloon catheter is advanced along the guide wire and then is fed into the hollow core holding the uncrimped stent. The stent then is loaded onto the balloon portion of the catheter.

In this alternative embodiment, a mandrel or a wire is inserted into the tool wilh the balloon catheter. The presence of the mandrel encourages the balloon catheter to maintain its shape during the crimping process. Without the mandrel, the crimp on the stent may be imperfect and accordingly, damage to the balloon catheter may result if the stent is crimped excessively. In an alternative embodiment, a mandrel may be provided as an integral component of the tool so that the mandrel extends from the tool, and the catheter can be guided onto the mandrel and into the correct position on each occasion the tool is used to crimp a stent.

When a stent has loaded onto the balloon portion of a catheter, a surgeon or a technician in a catheterization laboratory manually can yet precisely crimp the stent, using a tool embodying the present invention. Holding the tool in his or her hands, the surgeon can twist the distal section of the tool housing to accomplish the crimp.

The rotational motion of the distal section of the housing is translated into linear displacement or the screw-feed as follows: (1) the distal section and the proximal section are clipped together so as to permit relative rotation but to prevent relative linear displacement, (2) the collar is affixed to the proximal section so that it is prevented from rotating, and the threaded screw-feed likewise cannot rotate due to the linkage of the screw-feed with the collar through the teeth and plates; (3) the rotation of the distal section causes the screw-feed to rotate out of the distal section and simultaneously to advance linearly toward the proximal ead of the tool.

As this rotational motion occurs, the angular proximal edge of each tooth and plate is carried by the advancing screw-feed into engagement with the tapered end, resulting in contact and sliding displacement of the teeth and plates radially inward toward a central axis of the tool. In unison, the radiused edges of the teeth and plates converge upon the underlying stents thus crimping the stent onto the balloon catheter. The radiused edges of the plates act as crimping jaws.

Embodiments of the present invention also feature an optional visual indicator in order to assist the user in crimping the stent. More specifically, after the balloon catheter and the stent have been aligned, the distal section of the tool is twisted or screwed until a marking on the exterior of the distal section reaches a red line that previously has been painted on the exterior of a proximal section. The red line provides a visual cue to the user in response to which the use can stop twisting because the crimping step has been complete. Twisting the distal section beyond the red line creates a greater likelihood of damage to the balloon catheter due to excessive crimping.

After the crimping step has been accomplished, the user unscrews the distal section of the housing of the tool so that the balloon catheter and the crimped stent can slide freely through the passage in the tool. If the balloon catheter and the crimped stent do not slip easily through the crimping tool, then a repeat of the foregoing crimping procedure is indicated. For certainty, the process of testing the integrity of the crimp can be repeated until the user is assured that the crimped stent will pass safely through the crimping tool and to the patient.

Embodiments of the present invention represent an important step toward the handling of fragile stents. These embodiments significantly minimize the exposure of the user to stents that are radioactive by design, because the walls of the tool are preferably thicker than 6.25 millimeters (1/4 inch) so that the walls provide adequate shielding from the radioactive stent.

In addition, a radioactive stent can be packaged, shipped, and stored inside a crimping tool embodying the present invention for the length of the half-life of the radioactive stent. When the radioactive stent is to be implanted, the user simply loads the radioactive stent, which already is disposed within, the tool -- onto the balloon catheter, and then twists and untwists the tool housing to crimp and subsequently to release the stent, and finally feeds the crimped stent along the guide wire into the vasculature of the patient. Thus, a benefit of certain embodiments of the present invention are that the embodiment provide safe storage for the stent prior to implantation, while conveniently transforming into a crimping tool which does not require direct handling of the fragile and/or radioactive stent.

Tools embodying the present invention tool are versatile. First, the tool is ideal for handling stents because the tool has a larger diameter than the stent, but still is centered on the a ofthe stent. Accordingly, embodiment of the present invention allow clinicians to correctly crimp a stent onto a balloon.

Second, the tool effectively establishes a reliable stent crimp on a balloon of any diameter. This is possible because the tool has an opening that is approximately twice that of the diameter of the stent to accommodate the stent and the balloon catheter.

Third, the radiused edge of each tooth and plate of the tool can be designed to press the stent and the balloon catheter down to a specified diameter. This crimped dimension, of course, can be altered by changing the radius of the edges machined into the plates. Fourth, the tool is intended to be used with stents characterized by a variety of lengths. The total length of a tooth and plate in a preferred embodiment is over thirty-five millimeters long, thereby accommodating most, if not all, of the lengths of the stents currently on the market.

A crimping tool embodying the present invention can be used with any stent system which is released without a delivery system. Although the tool is intended to be a disposable device, the tool eventually may be sold as a reusable item because its design is robust enough to undergo many repeated uses.

In summary, a tool embodying the present invention protects clinicians from the dangers of beta (β) radiation that are emitted from radioactive stents. Also, the tool is capable of homogeneously and precisely crimping a stent onto a balloon catheter. Such a crimping tool is highly useful to cardiologists, for example. Such physicians always are concerned with proper deployment of the stent within the patient and thus find it desirable to have device which will result in a consistently and reliably crimped stent. The tool further is a time saver, because the stent crimping procedure can be performed fairly efficiently and quickly. These and other advantages of embodiments of the present invention will become apparent from the following detailed description thereof, when taken in conjunction with the accompanying exemplary drawings.

### BRIEF DESCRIPTION OF THE DRAWINGS

FIGURE 1 is a side elevational view, partially in section, depicting a stent that has been crimped onto a delivery catheter and disposed within a vessel.

FIG. 2 is a cross-sectional view of a preferred embodiment of a stent-crimping tool according to the invention, exposing the internal assembly of components.

FIG. 3 is a side elevational, exploded view of the stent-crimping tool shown in FIG. 2.

FIGS. 4A and 4B are cross-sectional and rear elevational views, respectively, of a proximal section of the housing of a preferred embodiment of the stent-crimping tool.

FIGS. 5A and 5B are front elevational and side elevational views, respectively, of a collar of a preferred embodiment of the stent-crimping tool showing the orthogonal slots formed in the collar.

FIGS. 6A and 6B are side elevational and front elevational views, respectively, of a distal section of the housing of a preferred embodiment of the stent-crimping tool.

FIGS. 7A, 7B, and 7C are top plan, front elevational, and side elevational views, respectively, of a tooth of a preferred embodiment of the stent-crimping tool having an angular proximal edge.

FIGS. 8A and 8B provide front elevational and side elevational views, respectively, of a screw-feed of a preferred embodiment of the stent-crimping tool.

### DETAILED DESCRIPTION OF THE PREFERRED EMBODIMENTS

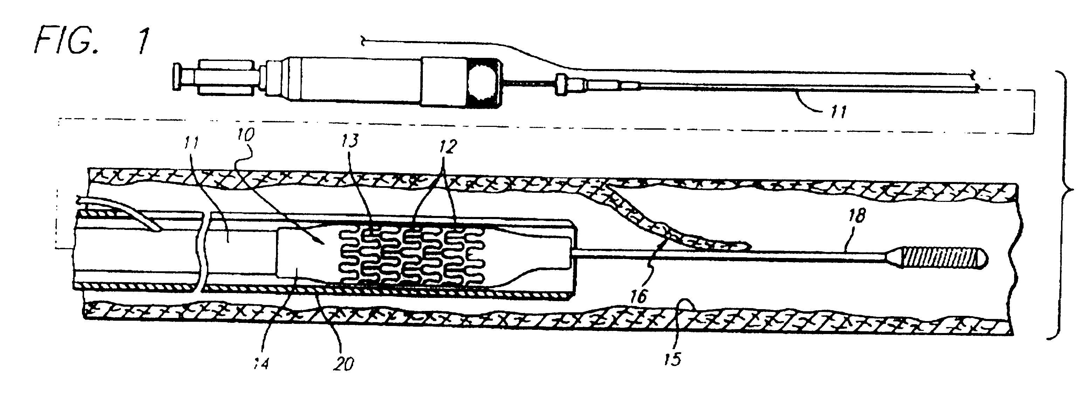

FIG. 1 illustrates an intravascular stent 10 which is mounted onto a delivery catheter 11. The stent 10 generally comprises a plurality of radially expandable cylindrical elements 12 disposed coaxially and interconnected by interconnections 13 disposed between adjacent cylindrical elements 12. The delivery catheter 11 has an expandable portion or a balloon 14 for expanding tbe stent 10 within a coronary artery 15 or other vessel such as saphenous veins, carotid arteries, arteries, and veins. The artery 15, as shown in FIG. 1, has a dissected lining 16 which has occluded a portion of the arterial passageway.

The delivery catheter 11 onto which the stent 10 is mounted essentially is the same as a conventional balloon dilatation catheter used for performing angioplasty procedures. The balloon 14 may be formed of suitable materials such as polyethylene, polyvinyl chloride, polyethylene terephthalate or other like polymers. In order for the stent 10 to remain in place on the balloon 14 during delivery to the site of the damage within the artery 15, the stent 10 is compressed onto the balloon 14.

An optional retractable protective delivery and alignment sleeve 20 may be provided to further insure that the stent 10 stays in place on the balloon 14 of the delivery catheter 11 and to prevent abrasion of the body lumen by the open surface of the stent 10 during delivery the desired arterial location. A removable, tubular-shaped carrier (not shown) might be used in place of, or in addition to, the sleeve 20 for the same purposes of security and preventing abrasion. Other means for securing the stent 10 onto the balloon 14 also may be used, such as collars or ridges provided on the ends of the working portion, i.e., the cylindrical portion, of the balloon 14.

In order to implant the stent 10, the stent first is mounted onto the inflation balloon 14 on the distal extemity of the delivery catheter 11. The stent 10 is crimped down onto the balloon 14 to insure a low profile. Embodiments of the present invention can be used to accomplish this crimping procedure.

The catheter-stent assembly can be introduced into the vasculature of a patient through processes known in the art. Briefly, a guide wire 18 is disposed across the arterial section where an angioplasty or atherectomy previously has been performed and where a stenting procedure now is indicated. In some cases, the arterial wall lining may be detached so that the guide wire 18 is advanced beyond the detached or dissected lining 16 and the catheter-stent assembly is advanced over the guide wire 18 within the artery 15 until the stent 10 is directly under the detached lining 16. Prior to inflation of the balloon 14, the delivery sleeve 20 is retracted to expose the stent 10. Depending upon characteristics of the balloon-and-stent assembly, a delivery sleeve may be unnecessary. The balloon 14 of the delivery catheter 11 then is inflated using an inflation fluid. Expansion of the balloon 14 in turn expands the stent 10 against the artery 15. Next, the balloon 14 is deflated and the catheter 11 is withdrawn, leaving the stent 10 to support the damaged arterial section. As mentioned above, in order to ensure proper seating of the stent to on the balloon 14, and to insure proper deployment of the stent 10 at the site of the damage within the artery 15, the stent crimping procedure is important.

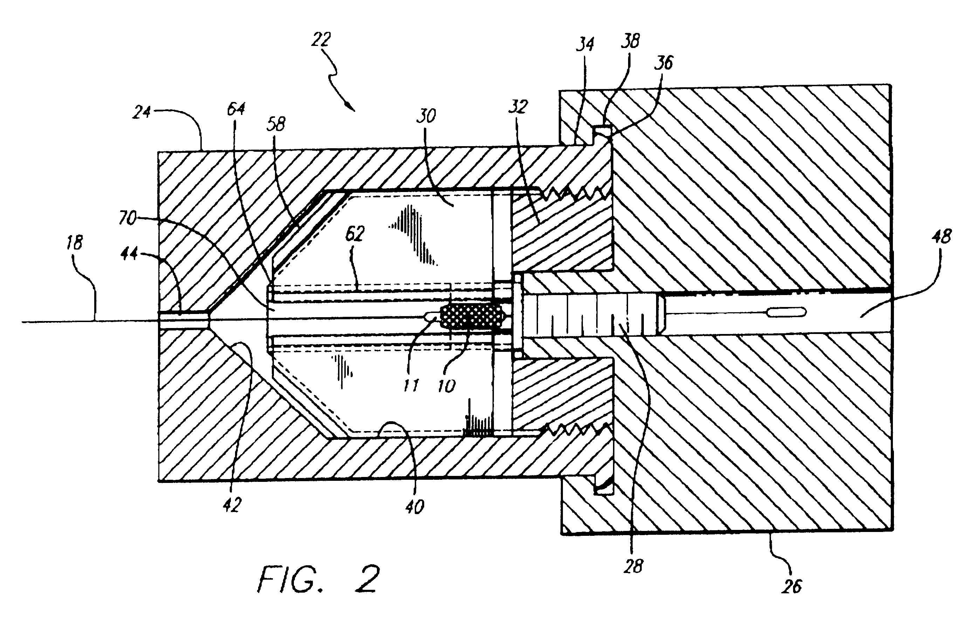

FIG. 2 is a sectional view of a preferred embodiment of the present invention stent-crimping tool 22. As can be recognized in this sectional view, the stent-crimping tool 22 is characterized by a preferably cylindrical housing which is comprised of two interlocking parts. Those interlocking parts are a proximal setion 24 and a distal section 26 of the housing. The two parts preferably are interconnected by inserting a portion of the proximal section 24 into a corresponding space 34 formed into the distal section 26, and whereby a lip 36 on the proximal section 24 becomes engaged within a groove 38 of the distal section 26. With this type of interconnection, relative rotation between the proximal section 24 and the distal section 26 is possible while relative linear displacement is limited insofar as the lip 36 is locked within the groove 38.

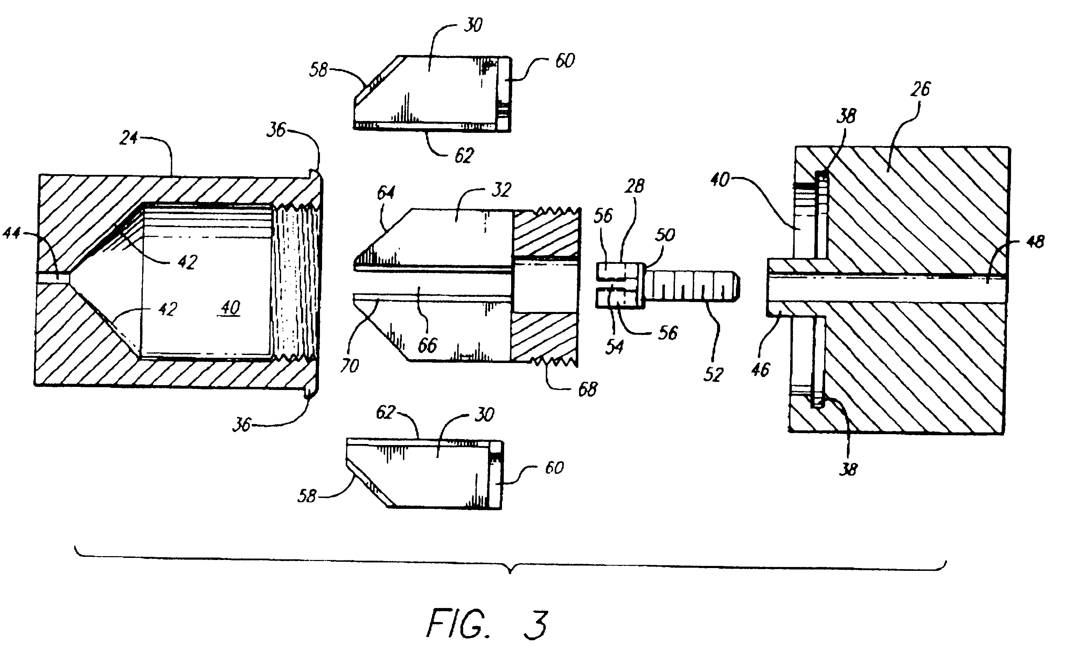

FIG. 2 thus provides a cross-sectional view of a preferred embodiment of the present invention in the assembled form of the stent-crimping tool. FIG. 3, on the other hand, is an exploded side elevational view of the major components a preferred embodiment of the present invention. In particular, FIG. 3 as well as FIG. 2 show the stent-crimping tool as being comprised of, moving from one end of the Figures to the opposite end of the Figures; a proximal section 24, teeth 30, a collar 32 a screw-feed 28, and a distal section 26. In FIG. 3, the intravascular stent 10, delivery catheter 11, and guide wire 18 have been omitted for clarity.

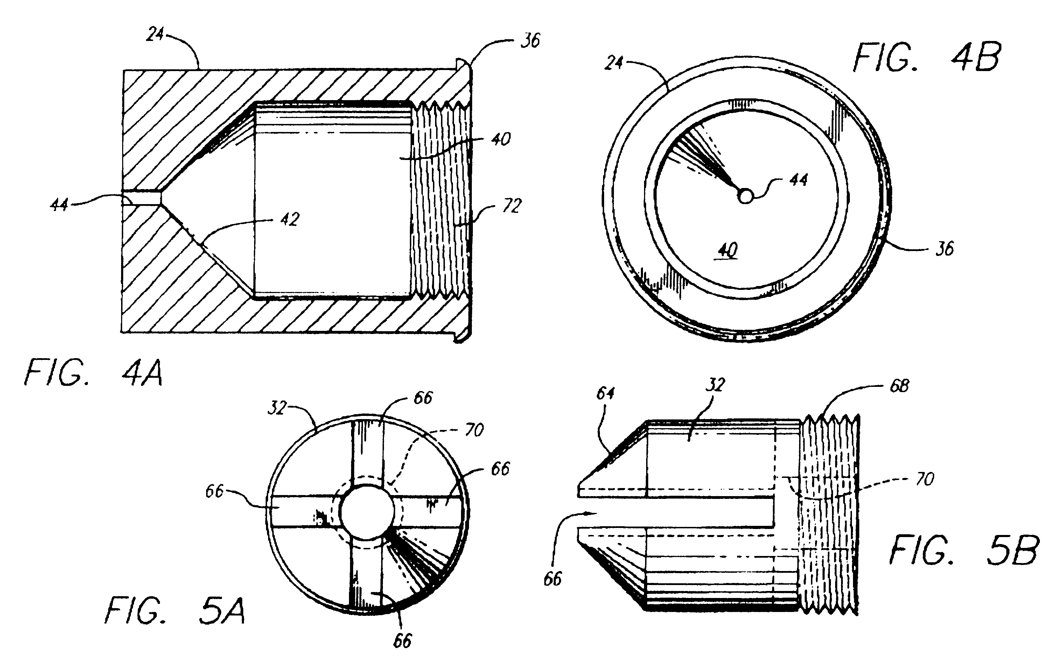

FIGS. 4A and 4B provide a sectional and a rear elevational view, respectively, of the proximal section 24. As seen in the drawings, the proximal section 24 preferably is cylindrical in shape having a cylindrical cavity 40, wherein the cylindrical cavity 40 includes a tapered end 42 leading to an opening 44. In the preferred embodiment, the tapered end 42 has a 45-degree taper from a central axis of the proximal section 24. Also seen in FIGS. 4A and 4B is the lip 36 which, as seen in FIG. 2, helps secure the proximal section 24 to the distal section 26. To facilitate assembly with the collar 32, the proximal section 24 has a 5-degree coarse thread 72 cut into the wall of the cylindrical cavity 40.

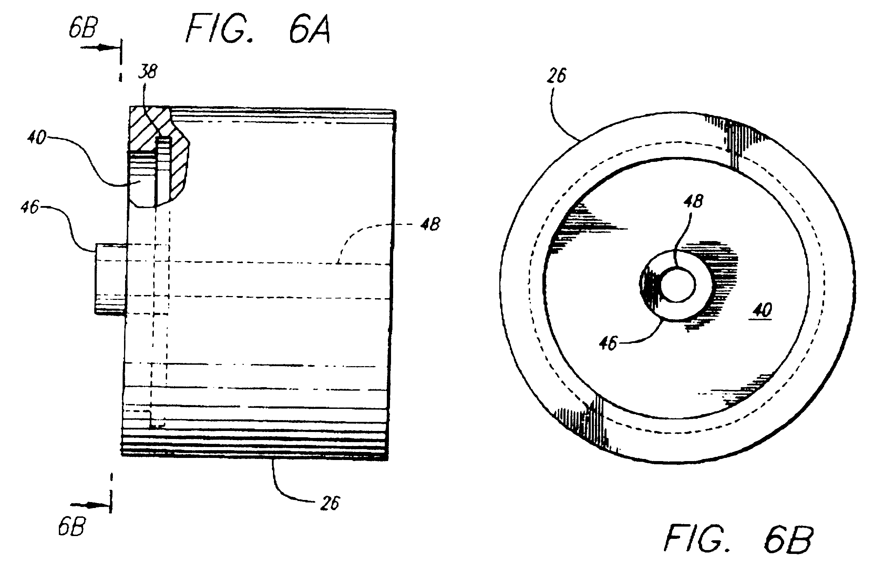

FIGS. 6A and 6B provide a side elevational and an end view, respectively, of the distal section 26 of the housing. In the preferred embodiment shown here, the distal section 26 has an ergonomic cylindrical shape with the cylindrical cavity 40 and the groove 38 used to catch the lip 36. The distal section 26 also includes a boss 46 with a tubular passage 48 extending therethrough. The end portion of the tubular passage 48 extending through the boss 46 is tapped to a 1/4"-20 threads to accept a threaded screw.

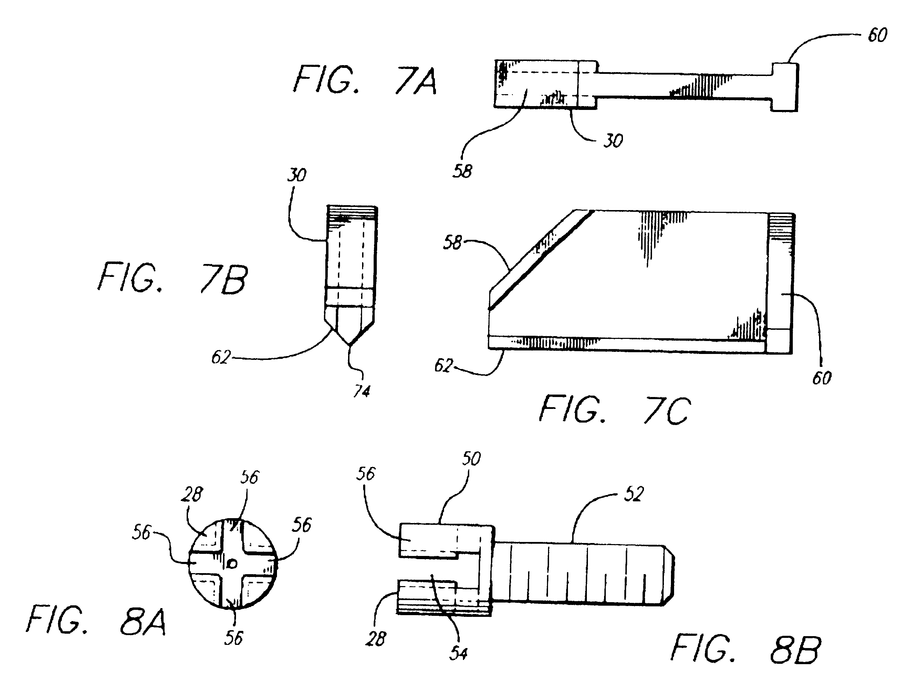

FIGS. 8A and 8B provide a front elevational and a side elevational view, respectively, of the screw-feed 28. As a device that translates rotational motion to linear motion, the screw-feed 28 is comprised of a head 50 and a shaft 52, the latter being cut with 1/4"-20 threads to match the corresponding threads formed in the boss 46 of the distal section 26.

The screw-feed 28 further includes a hollow core 54 that extends a length of the head 50 and the shaft 52. The hollow core 54 serves as a chamber to hold the stent 10. In addition, the screw-feed 28 includes preferably four orthogonal slots 56. These slots 56 are designed to receive and retain the teeth 30.

FIGS. 7A, 7B, and 7C provide a top plan, a front elevational, and a side elevational view, respectively, of a preferred embodiment a tooth 30. The tooth 30 essentially is a flat, almost trapezoidal shape plate having an angular proximal edge 58 formed at preferably 45 degrees in one corner of the plate.

Along the angular proximal edge 58 and long the distal edge 60 of the tooth are "T" formations. The "T" formation along the distal edge 60 of the tooth 30 conforms and slides into complementazy "T" cuts in the slots 56 of the screw-feed 28. A "T" formation is best seen in the distal edge 60 of FIG. 7A, and a corresponding "T" cut is best seen formed in the head 50 of FIG. 8B. The "T" formation along the angular maximal edge 58 of the tooth 30 conforms and slides into a complementary "T" cut formed in an orthogonal slot 66 of collar 32. As seen in FIG. 2, one function of the "T" formations is to slidably retain the teeth 30 in their respective slots 56 when the teeth 30 are assembled to the screw-feed 28.

The radiused edge 62 extends into the hollow core 54 of the screw-feed 28. As best seen in FIG. 7B, at the crest of the radiused edge 62 is a groove having a radius 74 that defines the final outside diameter of stent 10 after undergoing the crimping procedure. In a preferred embodiment, this radius 74 is in the order of 0.007 inch. Needless to say, changing the size or shape of the radius 74 changes the final outside diameter of the crimped stent.

In an alternative embodiment (not shown), the radiused edge may have a contour. The contour from a cross-sectional point of view, either axially or radially, may have a rectangular, arcuate, diamond, saw tooth, sinusoidal, ridged, or like profile known in the art. Such radiused edges help improve stent retention.

FIGS. 5A and 5B provide a front elevational and a side elevational view of a preferred embodiment of the collar 32. As best seen in those drawings, the collar 32 has a generally cylindrical shape with a conical end 64 angled at approximately 45 degrees from a horizontal axis. Accordingly, the conical end 64 and the cylindrical body of the collar 32 are adapted to fit witbin the tapered end 42 and the cylindrical cavity 40.

As mentioned earlier, four orthogonal slots 66 are formed into the collar 32 through which four teeth 30 pass. The angular proximal edge 58 of each tooth 30 is retained in the collar 32 by "T" formations formed along the angular proximal edge 58 sliding inside "T" cuts formed in the orthogonal slots 66.

At a distal end of the collar 32 are 5-degree coarse threads 68 intended to match the threads 72 in the proximal housing 24. Threads 68 and 72 in the collar 32 immovably inside the cylindrical cavity 40 of the proximal section of the housing 24. The collar 32 further includes a tubular passage 70 in communication with the opening 44 in the cylindrical cavity. The passage 70 extends the entire length of the collar 32.

All together, the foregoing structures are assembled according to that shown in FIGS. 2 and 3. More precisely, the teeth 30 are slid into the corresponding slot 66 of the collar 32 and therefore extend into the slots 56 formed in the head 50 of the screw-feed 28. Thus the joined, screw-feed 28 cannot rotate because it is linked via the teeth 30 to the collar 32, which is tightly screwed into the proximal section 24.

The head 50 of the screw-feed 28 extends into the passage 70 within the collar 32 while the threaded shaft 52 is screwed into the boss 46 of the distal section 26 of the housing. The uncrimped intravascular the stent 10 is placed in the hollow core 54. The major pieces of the housing are assembled as shown in FIG. 2 so that the lip 36 latches or snaps into the groove 38 while the collar 32 is immovably mounted to the cylindrical cavity 40 of the proximal section 24 of the housing.

The next step is to load the stent 10 onto the delivery catheter 11. This is accomplished by inserting a guide wire 18 into the opening 44 of the proximal section 24 and through the passage 70 of the collar 32, extending out the hollow core 54. The tool 22 can then be advanced along the guide wire 18. The balloon catheter 11 and a mandrel (not shown) are advanced over the guide wire 18 into the passage 70. In an alternative embodiment, the mandrel may be built into the tool so that it extends therefrom, and the catheter is guided onto the mandrel and into the correct position every time.

The user then manually aligns the balloon 14 with the uncrimped stent 10 inside the hollow core 54. Alignment is possible when the key structures of the tool 22 are formed from a transparent or translucent material so that the user can see the location of the two parts. Other alignment methods known in the art can be employed as well.

The crimping step is next. The user holds the proximal section 24 of the housing while twisting the distal section 26 of the housing. Because their relative linear motion between these two elements is held constant, the relative rotational movement of the proximal section 24 and the distal section 26 causes the screw-feed 28 to advance out of the boss 46. As described above, the screw-feed 28 does not rotate relative to the proximal section 24 of the housing, due to the presence of the teeth 30 extending between the head 50 and the immobilized collar 32.

As the screw-feed 28 advances toward the proximal section 24, it carries forward the teeth 30 so that the angular proximal edges 58 of each tooth 30 encounter the tapered end 42, which in turn forces the teeth 30 to converge radially inward. As this convergence occurs, the radiused edges 62 of the teeth 30 engage and crimp the underlying stent 10 onto the balloon catheter 11. The teeth 30 thus act as jaws closing down on the stent 10. The mandrel optionally lorded into the delivery catheter 11 prevents the crimping process from overly compressing the stent 10 onto the catheter 11.

Rotating the distal section 26 of the housing in the opposite direction moves the screw-feed 28 in a direction away from the tapered end 42 of the proximal section 24 ofthe housing. As a result, pressure from the radiused edges 62 of the teeth 30 is removed from the crimped stent 10. Thereafter, the user may push the crimped stent and catheter assembly freely through the passage 70, so that the combination appears outside of the tool 22 for subsequent implantation in a patient. Of course, the foregoing steps for crimping the stent can be repeated as necessary.

Optional markings (not shown) on an exterior surface of the proximal section 24 or the distal section 26 of the housing may assist the user in determining the optimum amount of twisting needed to achieve a particular crimp. The markings can be calibrated at the factory for a given size or type of stent.

The shape of particular embodiments of the tool may be changed to offer improved ergonomics for the user. It is expected that the tool is machined from acrylic or lexan. Later versions of the tool may be created from alternative materials that offer comparable shielding from a radioactive source.

Moreover, the radiused edges 62 of the teeth 30 may be coated or covered with a protective material. The radiused edge 62 may be polished as well so that it does do little harm to the polished finished of the stent 10. In alternative embodiments, there may be more or fewer than four teeth 30 as described above. Naturally, the number of slots in the collar 32 and the screw-feed 28 and their angular placement surrounding the stent should be modified as necessary, and the are defining the radiused edge of each tooth should be modified as well.

Embodiments of the tool are sterilized and intended to be used in a catheterization laboratory by a trained technician or cardiologist. More precisely, and as will be appreciated by those skilled in the art, the crimping tool 22 is designed both for single use applications in a catheterization laboratory by a physician and for multiple use applications in a sterile environment in a high-volume manufacturing facility. In such a manufacturing facility, where sterile conditions exist, the crimping tool 22 can be used repeatedly to crimp stents onto balloons until the mechanism wears out. Thus, repeated uses of the tool are contemplated for controlled, sterile environments, as are single use applications when operated by catheterization laboratory personnel.

Further, embodiments of the crimping tool can be used with any stent that is released without a delivery system. The crimping tool also may be sold alone, because its design is robust enough to undergo many uses.

Other modifications can be made to the present invention without departing from the scope thereof. The specific dimensions, procedural steps, and materials of construction are provided as examples, and substitutes readily are contemplated which do not depart from the invention.

## Claims

1. A tool (22) for crimping a stent (10) onto a balloon catheter (11), comprising:
a housing including a proximal section (24) and a distal section (26), wherein the proximal section (24) includes a cylindrical cavity (40) having a tapered end (42) and an opening (44) in communication with the cylindrical cavity;
a cylindrical collar (32) having a conical end (64) conforming to the tapered end (42) of the cylindrical cavity (40) when positioned therein, wherein the collar (32) includes a plurality of slots (66) extending radially and along a length of and intersecting at the conical end (64), and having a page (70) therethough in commmunication with the opening (44) in the proximal section (24);
a plurality of teeth (30), each tooth (30) having an angular proximal edge (58), and being slidably disposed inside a corresponding slot (66) in the collar (32), each tooth (30) having a radiusod edge (62) extending into the passage (70); and
a screw-feed (28) extending into the passage (70) wherein the screw-feed includes radial slots (56) that lead into a hollow core (54), and the slots (56) receive the respective radiused edges (62) of the teeth (30) extending therein, and wherein the screw is threaded to the distal section (26) of the housing;
whereby an uncrimped stent (10) loaded onto the balloon catheter (11) is located in the hollow core (54), whereby rotating the distal section (26) of the housing advances the screw and the angular proximal edge (58) of the teeth (30) into the tapered end (42) thereby displacing the teeth (30) radially inward so that the radiused edges (62) of the teeth (30) crimp the stent (10) onto the balloon catheter (11).

2. The crimping tool (22) of claim 1, wherein the radiused edge (62) includes a radius that approximates an outside diameter of a crimped stent.

3. The crimping tool (22) of claim 1, wherein the tool is composed in whole or in pall of a translucent polymer.

4. The crimping tool (22) of claim 1, wherein tool is composed in whole or in part of a polymer selected from the group of polymers consisting of acrylic or lexan.

5. The crimping tool (22) of claim 1, wherein the tool includes a mandrel disposed within the balloon catheter (11).

6. The crimping tool (22) of claim 1, wherein the collar (32) includes four slots (66) disposed radially at right angles.

7. The crimping tool (22) of claim 1, wherein the collar (32) is affixed to the housing.

8. The crimping tool (22) of claim 1, wherein the distal section (26) of the housing includes a groove (38) to receive a lip (36) formed in the proximal section (24) of the housing, to facilitate relative rotational motion only.

9. The crimping tool (22) of claim 1, wherein the housing includes reference markings on an exterior surface.

10. The crimping tool (22) of claim 1, wherein the radiused edge (62) of each tooth (30) is contoured.

11. A tool (22) for crimping a stent (10) onto a balloon catheter (11), comprising:
a housing including a proximal section (24) rotatably attached to a distal section (26), wherein the proximal section (24) includes a cylindrical cavity (40) having a tapered end (42), and an opening (44) in communication with the cylindrical cavity;
a cylindrical collar (32) affixed to the proximal section (24) of the housing having a conical end (64) approximating the shape of the tapered end (42), wherein the collar (32) includes a plurality of slots (66) extending radially and along a length thereof and intersecting at the conical end (64), and having a cylindrical passage (70) therethrough in communication with the opening (44) in the housing;
a plurality of teeth (30), each tooth (30) having an angular proximal edge (58) approximating the shape of the tapered end (42), and being slidably disposed inside a corresponding slot (66) in the collar (32), each tooth (30) having a radiused edge (62) extending into the cylindrical passage (70); and
a screw-feed (28) extending into the cylindrical passage (70) and threaded to the distal section (26) of the housing, and wherein the screw-feed (28) includes a head (50) with hollow core (54) that is in communication with the cylindrical passage (70), and the head (50) further includes radial slots (56) aligned with the slots (66) in the collu (32) such that the radiused edges (62) of the teeth (30) extend through the slots into the hollow core (54);
whereby an uncrimped stent (10) loaded onto the balloon catheter (11) is positioned in the hollow core (54), whereby routing the distal section (26) of the housing advances the screw-feed (28) and the angular proximal edge (58) of the teeth (30) into the tapered end (42) thereby displacing the teeth radially inward so that the radiused edges (62) of the teeth (30) crimp the stent (10) onto the balloon catheter (11).

12. The stent crimping tool (22) of claim 11, wherein the housing is characterized by a cylindrical exterior.

13. The stent crimping tool (22) of claim 11, wherein the radiused edges (62) of the teeth (30) are coated with a material selected from the group consisting of teflon, a lubricious polymer, or a biocompatible coating.

14. The stent crimping tool (22) of claim 11, wherein the tool is composed in whole or in part of a material that absorbs radiation.

15. A method of crimping a stent (10) onto a ballon catheter (11) comprising the steps of;
providing a housing including a proximal section (24) rotatably mounted to a distal section (26), wherein the proximal section (24) includes a cylindrical cavity (40) having a tapered end (42) with an opening (44);
providing a cylindrical collar (32) having a conical end (64), wherein the collar (32) includes a plurality of slots (66) extending radially and along a length thereof and intersecting at the conical end (64), and having a cylindrical passage (70) therethrough in communication with the opening (44);
mounting the cylindrical collar (32) to the proximal section (24) so that the conical end (64) conforms with the tapered end (42);
providing a plurality of teeth (30), each tooth (30) having an angular proximal edge (58), and being slidably disposed inside a corresponding slot (66) in the collar (32), each tooth (30) having a radiused edge (62) exuding into the passage (70);
providing a screw-feed (28) extending into the cylindrical passage (70) and threaded to the distal section (26) of the housing, and wherein the screw-feed (28) includes a head (50) with a hollow core (54) that is in communication with the cylindrical passage (70), and the head (50) further includes radial slots (56) aligned with the slots (66) in the collar (32) such that the radiused edges (62) of the teeth (30) extend through the slots into the hollow core (54);
placing an uncrimped stent (10) in the hollow core (54) of the screw-feed (28),
advancing a balloon catheter (11) through the passage (70) into the hollow core (54);
loading the stent (10) onto the balloon catheter (11); and
rotating the distal section (26) of the housing to advance the screw-feed (28) and the angular proximal edge (58) of the teeth (30) into the tapered end (42) thereby displacing the teeth radially inward so that the radiused edges (62) of the teeth crimp the stent (10) onto the balloon catheter (11).

16. The method of claim 15, wherein the collar (32), teeth (30), and housing are composed in whole or in part of a transparent polymer so that the crimping step is visible.

17. The method of claim 15, further comprising the step of advancing a guide wire (18) through the owning (44), the cylindrical passage (70), and the hollow core (54) holding the stent (10).

18. The method of claim 15, further comprising the step of inserting a mandrel inside the balloon catheter (11).

19. The method of claim 15, wherein the distal section (26) of the housing characterized by a cylindrical exterior.

20. The method of claim 15, further comprising the steps of (1) providing markings on the exterior of the proximal section (24) and distal section (26) of the housing; and (2) rotating the distal section (26) until the markings are aligned.

21. The method of claim 15, wherein a mandrel is provided in the hollow core (54), and further comprising the step of centering the hollow core (54) holding the stent (10) on the mandrel.

22. The method of claim 15, wherein the radiused edge (62) of each tooth (30) has a contour.
